**Europäisches Patentamt**

**European Patent Office**

**Office européen des brevets**

(19)

(11) Numéro de publication: **0 230 856**
**B1**

(12) # FASCICULE DE BREVET EUROPEEN

(45) Date de publication du fascicule du brevet:
03.10.90

(51) Int. Cl.⁵: **A61B 17/56**

(21) Numéro de dépôt: **86810535.4**

(22) Date de dépôt: **24.11.86**

(54) Fiche transcutanée de fixation d'un fragment ou élément osseux.

(30) Priorité: **28.11.85 CH 5070/85**
**13.10.86 CH 4071/86**

(43) Date de publication de la demande:
**05.08.87 Bulletin 87/32**

(45) Mention de la délivrance du brevet:
**03.10.90 Bulletin 90/40**

(84) Etats contractants désignés:
**AT BE CH DE ES FR GB GR IT LI LU NL SE**

(56) Documents cités:
**FR-A- 2 517 535**
**US-A- 2 388 482**
**US-A- 2 393 982**
**US-A- 4 414 966**
**US-A- 4 537 185**

(73) Titulaire: **JAQUET ORTHOPEDIE S.A., 5, chemin des Aulx, CH-1228 Plan-les-Ouates(CH)**

(72) Inventeur: **Wagenknecht, Marcel, 12, chemin des Milans, CH-1219 Le Lignon(CH)**

(74) Mandataire: **Dietlin, Henri et al, Dietlin & Cie S.A. Rue des Epinettes 19, CH-1227 Genève(CH)**

ACTORUM AG

## Description

Les fiches transcutanées sont connues depuis de nombreuses années et sont utilisées dans la chirurgie osseuse, notamment dans l'ostéosynthèse externe. Ces fiches traversent la chair et les os et sont, en général, utilisées avec des appareils de fixation squelettique externe développés par le Dr. Hoffmann. Ces appareils de fixation squelettique externe comprennent des rotules, des barres de liaison, des barres coulissantes, des articulations et des étaux d'ancrage destinés à retenir et positionner les fiches transcutanées. Les fixateurs externes d'Hoffmann consistent en un cadre de montage placé autour d'un membre ou d'une partie du corps humain, ce cadre étant agencé pour maintenir depuis l'extérieur des os ou fragments d'os à l'aide de fiches ou de groupes de fiches transcutanées.

Il existe actuellement sur le marché un grand nombre de types de fiches transcutanées. Ces fiches comprennent généralement une partie filetée avec à leur extrémité une partie taraudante agencée pour effectuer un filetage lorsqu'elle est vissée dans un fragment ou élément d'os. Avant la mise en place d'une telle fiche, l'os ou le fragment d'os a été préalablement percé au diamètre voulu, lequel est sensiblement égal ou supérieur au diamètre intérieur du filetage. L'extrémité de la fiche est alors introduite dans le trou percé et la partie filetée de la fiche est vissée à l'intérieur du trou. Lors de cette opération, le taraud prévu à l'extrémité de la fiche effectue le taraudage du trou préalablement percé. Pour visser la fiche dans le trou, on utilise habituellement un mandrin venant s'introduire dans une partie à section polygonale, par exemple une section carrée située à l'une des extrémités de la fiche. Dans les fiches de l'art antérieur, l'extrémité de la partie filetée peut se présenter comme l'extrémité d'une mèche de perçage ou présenter différentes configurations connues de l'homme de métier.

Il existe également un type de fiche, appelée fiche transfixiante, dans laquelle la partie filetée est précédée d'une tige présentant à son extrémité libre une pointe de perçage. Les fiches transfixiantes peuvent être mises en place sans prévoir préalablement dans l'os un perçage. Dans la fiche transfixiante, le perçage sera effectué par l'extrémité de la pointe et le taraudage par une partie taraudante prévue au début du filetage. Des fiches transcutanées et transfixiante du type décrit ci-dessus sont connues de US-A 4 537 185, US-A 2 388 482 et FR-A 2 517 535.

Les fiches transcutanées de l'art antérieur ne permettent pas d'obtenir un perçage ou un taraudage de très bonne qualité. Comme dans toute opération de perçage ou de taraudage, la température augmente à l'endroit du perçage ou du taraudage, ce qui peut créer des nécroses et des détériorations dans les tissus osseux. D'autre part, les fiches de l'art antérieur ne permettent pas d'obtenir des surfaces de taraudage de bonne qualité. Ces surfaces présentent des parties arrachées qui ne permettront pas par la suite d'obtenir une bonne qualité d'ancrage entre le fragment d'os et la fiche lorsque cette dernière est mise en place. Finale-ment, les fiches de l'art antérieur, de par leur mauvais fonctionnement quant à l'opération de perçage ou de taraudage, peuvent provoquer lorsqu'elles sont en place des contraintes exagérées dans l'os.

Le but de la présente invention est de proposer une fiche transcutanée assurant un perçage ou un taraudage de bonne qualité, tout en garantissant une augmentation minimale de la température lors de l'insertion.

La fiche transcutanée de fixation d'un fragment ou élément osseux selon l'invention est caractérisée par les caractéristiques définies dans la revendication 1.

La présence du dégagement de dépouille permet un taraudage de bonne qualité et une pénétration plus facile de la fiche dans l'os. De plus, étant donné que les copeaux ne restent pas à l'intérieur de l'os durant l'opération et ne forment ainsi pas obstacle pour l'arête de coupe suivante, l'augmentation de température est beaucoup moins élevée qu'avec les fiches de l'art antérieur.

La partie avant du filetage de la fiche peut se présenter sous forme d'un cône d'entrée, les goujures définissant les arêtes de coupe s'étendant sur le cône d'entrée du filetage et sur le début de la partie centrale cylindrique du dit filetage.

Différentes variantes peuvent être prévues pour réaliser les goujures formant l'arête de coupe. Les goujures peuvent se présenter sous forme d'une encoche formée de deux surfaces sensiblement perpendiculaires ou sous forme d'une encoche de section générale arrondie. Les pas de l'hélice et du filetage tournent dans le même sens. Les goujures sont réalisées par meulage, après que le filetage avec son cône d'entrée ait été réalisé.

L'invention s'adapte aux différents types de fiches, qu'il s'agisse de fiches nécessitant un préperçage ou de fiches auto-perforantes. Dans ce dernier cas, la fiche présentera avantageusement une pointe de perçage placée au moins indirectement en début du cône d'entrée du filetage.

L'invention comprend également une fiche transfixiante, dans laquelle le cône d'entrée du filetage est précédé d'une tige de diamètre égal ou plus faible que le diamètre de fond de filet de la partie filetée, la tige se terminant par une pointe plate de perçage en forme d'ogive. La pointe plate en forme d'ogive peut présenter à sa partie supérieure deux saignées de dégagement placées en regard l'une de l'autre.

La pointe plate en forme d'ogive peut présenter une section en forme de parallélogramme allongé définissant deux surfaces de coupe opposées, chacune suivie d'une surface formant un angle de dépouille, les dites surfaces de coupe et de dépouille formant ensemble une arête de coupe, les surfaces de coupe présentant un évidement allongé sous forme de cuillère placé adjacent à l'arête de coupe, de manière à modifier l'angle de coupe pour l'amener à zéro ou vers une valeur positive.

Le dessin annexé représente, à titre d'exemples, différents modes d'exécution de la fiche transcutanée objet de l'invention.

Dans le dessin:

La fig. 1 est une vue latérale représentant un premier mode d'exécution d'une fiche transcutanée,

la fig. 2 est une vue latérale représentant un deuxième mode d'exécution d'une fiche transcutanée, appelée fiche transfixiante,

la fig. 3 est une vue agrandie de l'extrémité de la fiche représentée dans la fig. 1 selon la vue III, dans une première forme d'exécution de fiche mise en place après que l'os ait été percé à un diamètre adéquat; cette fiche est souvent appelée demi-fiche pointe mousse;

la fig. 4 est une vue similaire à la fig. 3, dans une deuxième forme d'exécution de fiche transfixiante, permettant d'effectuer de plus le perçage avant de réaliser le taraudage et connue sous la dénomination de demi-fiche auto-perforante et auto-taraudante,

la fig. 5 est une vue agrandie de l'extrémité avant du filetage d'une fiche transfixiante, dans une première variante d'exécution,

la fig. 6 est une vue similaire à la fig. 5, dans une seconde forme d'exécution,

la fig. 7 est une coupe selon les lignes VII-VII de la figure 5,

la fig. 8 est une coupe selon les lignes VIII-VIII de la figure 6,

la fig. 9 est une vue de détail agrandie de la pointe de la fiche de la fig. 2, selon la flèche IX, et la fig. 10 est une coupe à travers la pointe de la fig. 9, selon la ligne X-X.

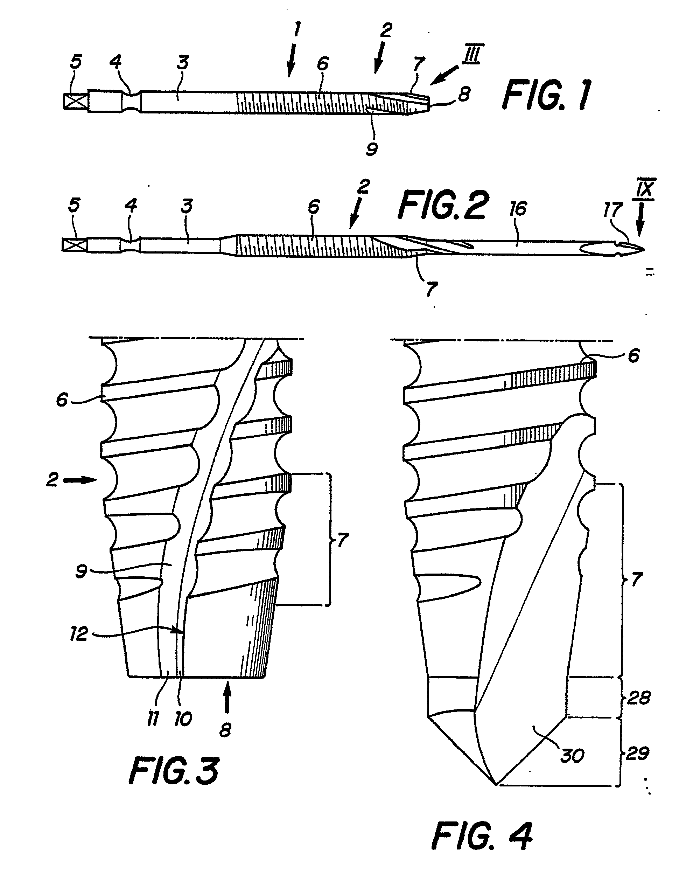

La fiche transcutanée 1 représentée dans les fig. 1 et 3 comprend une partie filetée 2 qui est la partie avant de la fiche et une partie arrière que nous appellerons partie lisse 3. La fiche 1 est une fiche cylindrique de faible diamètre par rapport à sa longueur et la partie arrière ou partie lisse 3 présente une rainure 4 et une partie d'extrémité à quatre pans 5. La partie d'extrémité 5 permettra l'introduction d'un mandrin qui servira à entraîner la fiche 1 dans un fragment ou élément osseux tandis que la rainure 4 permet de retenir ce mandrin. La partie avant 2 de la fiche 1 (voir en particulier fig. 3) comprend un filetage 6 dont la partie avant se présente sous forme d'un tronc de cône d'entrée 7 terminé par une face sensiblement plane 8, perpendiculaire à l'axe de la fiche.

Aux figures 3, 5 et 7, trois goujures 9 sont placées à 120° l'une par rapport à l'autre sur le pourtour de l'extrémité avant de la fiche, de manière à permettre l'opération de taraudage. Ces goujures 9 se présentent sous forme de deux surfaces perpendiculaires 10 et 11, la surface 10 définissant à son extrémité libre l'arête de coupe 12 du taraud (voir également la coupe de la fig.7). Chacune des arêtes de coupe 12 peut être suivie dans le sens de rotation de la fiche par un dégagement de dépouille 13 allant en se rapprochant de l'axe de la fiche jusqu'à son intersection avec la surface 11 de la goujure suivante 9.

Les goujures 9 sont de préférence réalisées par meulage. L'opération de meulage est réalisée à l'aide d'une meule circulaire déplacée tangentiellement sur l'extrémité du filetage 6. La réalisation des goujures 9 par meulage donne une bonne qualité de surface à l'arête de coupe et permet d'éviter une opération d'ébavurage ultérieure. La réalisation du dégagement de dépouille 13 se fait de la même manière en approchant une meule de l'extrémité de la fiche en faisant tourner celle-ci tout en rapprochant la meule de l'axe de la fiche.

Bien que la réalisation des goujures 9 et du dégagement de dépouille ait donné de très bons résultats par meulage, il est évident pour l'homme du métier qu'elle peut également être faite par fraisage ou par tout autre moyen adéquat.

Le type de fiche présenté à la figure 3 est plus particulièrement utilisé pour être placé dans des os dans une position telle que, à la sortie du second cortex, la pointe de la fiche ne risque pas de blesser des tissus à proximité de l'os.

Par contre, dans d'autres utilisations, il peut être intéressant de mettre en place une fiche dite transfixiante, c'est-à-dire une fiche successivement auto-perceuse et auto-taraudeuse, telle celles représentées aux figures 2 et 4.

A la figure 4, comme dans la description précédente, la fiche comporte un filetage 6 se terminant sur une partie tronc-cônique 7. Elle se différencie néanmoins en ce que le tronc de cône 7 est prolongé par une partie cylindrique 28, elle-même terminée par une pointe de forme générale cônique 29. La partie cylindrique 28 permet d'éviter, dans un os cortical d'épaisseur normale, une réaction du perçage sur le taraudage rendant difficile la réalisation correcte d'un filetage dans l'os. Dans cette exécution, la fiche comporte deux goujures symétriques 30.

En variante, on peut prévoir une fiche transfixiante telle que représentée à la figure 2, et qui comprend comme la fiche de la fig. 1 une partie filetée 2, une partie arrière 3 à l'extrémité de laquelle est prévue une rainure annulaire 4 et une partie d'extrémité 5 à quatre pans destinée à être reçue dans un mandrin d'entraînement.

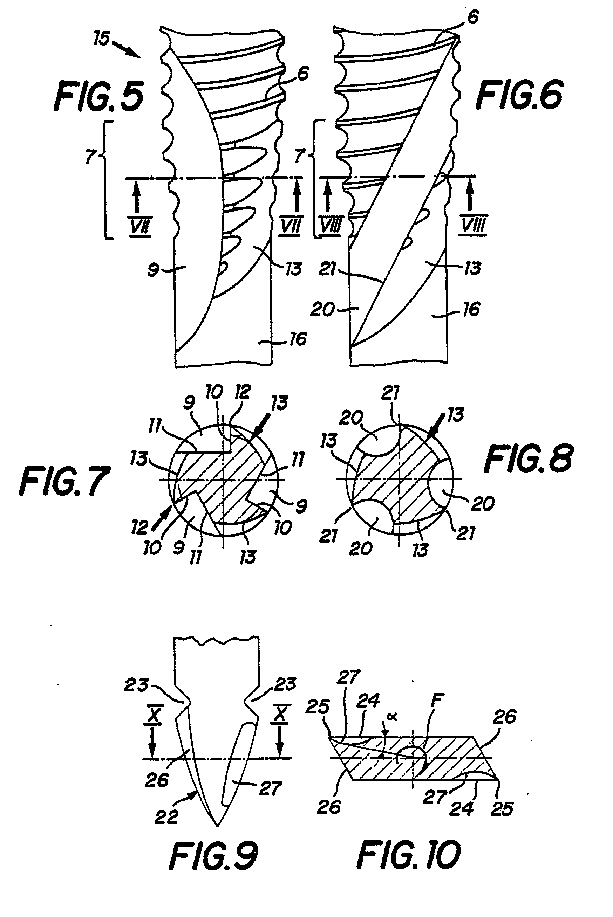

La partie filetée 2 comprend un filetage 6 avec un cône d'entrée 7 similaire à celui détaillé à la figure 3. Ici encore les goujures de taraudage 9 peuvent être réalisées au moyen d'une meule circulaire qui sera déplacée tangentiellement à l'axe de la fiche, en partant de la tige 16 située à l'avant du tronc de cône 7, pour déboucher sur le filetage 6. La tige 16 est terminée vers l'avant par une pointe de coupe 17 destinée à effectuer le perçage, qui est présentée plus en détail à la figure 9.

La pointe 17 placée à l'extrémité de la tige 16 de la fiche transfixiante représentée dans la fig. 2 se présente sous forme d'une ogive 22, l'ogive ayant à sa partie supérieure deux saignées de dégagement 23 (fig. 9). La pointe en forme d'ogive 22 présente des caractères constructifs particulièrement intéressants qui sont visibles sur la fig. 9 et sur la coupe de la fig. 10 qui montre une configuration en forme de parallélogramme allongé comprenant deux surfaces de coupe 24 définissant une arête de coupe 25 suivie d'une surface de dépouille 26. Si l'on fait tourner la pointe dans le sens de rotation représenté par la flèche F, on remarque que les surfaces 26 définissent un angle de dépouille suffisamment important pour permettre le dégagement des copeaux obtenus lors du perçage d'un os au moyen de l'ogive 22. Par contre, l'homme du métier remarquera dans la fig. 9 que les surfaces planes 24, définissent des surfaces de coupe avec un angle de coupe alpha négatif. Pour modifier cet angle de coupe alpha et l'amener à 0 ou vers une valeur positive, il a été prévu deux évidements allongés 27 placés adjacents à l'arête de coupe 25. Ainsi, l'angle de coupe alpha peut être corrigé et choisi en fonction des désirs de l'utilisateur en modifiant la profondeur des évidements 27.

La pointe des fig. 9 et 10 avec sa forme en ogive, ses surfaces de dépouille 26 et ses évidements 27 ramenant l'angle de coupe vers 0 ou vers une valeur positive, permet d'effectuer des perçages de très bonne qualité par rapport aux fiches de perçage de l'art antérieur. La pointe en forme d'ogive est d'autre part particulièrement adéquate, car elle se fiche dans l'os et permet d'effectuer un perçage selon un axe sans aucune déviation latérale.

La fiche qui vient d'être décrite en regard des figures 1, 3 et 4 présente une longueur totale qui peut varier entre 75 et 200 mm pour des diamètres allant de 3 à 6 mm. Généralement, le tronc de cône présente une inclinaison de 12°.

La fiche représentée selon le mode d'exécution de la fig. 2, avec une partie filetée centrale, aura une longueur totale allant de 175 à 350 mm pour des diamètres allant également de 3 à 6 mm. La partie avant de la fiche représentée dans la fig. 2 consistant en la tige 16, aura un diamètre égal ou plus faible que le diamètre de fond de filet de la partie filetée 6. Pour une fiche de 3, respectivement 4, 5 et 6 mm, la partie 16 aura respectivement 2, 3, 4 et 5 mm. Dans le cas de la fiche de la figure 2, on a avantage à prévoir un tronc de cône d'entrée de 6°.

Dans les différents exemples donnés, le filetage comporte un filet par tour, mais il est bien sûr possible de prévoir en variante davantage de filets par tour, les tarauds passant successivement plusieurs fois dans la même gorge de taraudage.

**Revendications**

1. Fiche transcutanée de fixation d'un fragment ou élément osseux comprenant une partie filetée dont la partie avant se présente sous forme d'un tronc de cône d'entrée et au moins une goujure définissant une arête de coupe s'étendant sur ledit tronc de cône d'entrée du filetage et sur le début de la partie centrale cylindrique dudit filetage pour effectuer une opération de taraudage lorsque l'extrémité de la partie filetée pénètre dans le fragment ou l'élément osseux, caractérisée en ce que la/les goujure(s) est/sont disposée(s) hélicoïdalement par rapport à l'axe du filetage, les pas de l'hélice et du filetage tournant dans le même sens.

2. Fiche selon la revendication 1, caractérisée en ce qu'elle comporte au moins deux goujures régulièrement disposées sur la circonférence de la fiche.

3. Fiche selon la revendication 1 ou 2, caractérisée en ce que la goujure se présente sous forme d'une encoche formée de deux surfaces sensiblement perpendiculaires.

4. Fiche selon la revendication 1 ou 2, caractérisée en ce que la goujure se présente sous forme d'une encoche de section de forme générale arrondie.

5. Fiche selon la revendication 1 ou 2, caractérisée en ce que la partie avant dudit tronc de cône se finit par un plat perpendiculaire à l'axe de la fiche et destiné à s'engager dans un perçage pratiqué préalablement dans l'élément osseux.

6. Fiche selon la revendication 5, caractérisée en ce que l'arête entre ledit plat et le tronc de cône est cassée.

7. Fiche selon la revendication 1 ou 2, caractérisée en ce que le tronc de cône d'entrée du filetage est précédé d'une tige de diamètre égal ou plus faible que le diamètre de fond de filet de la partie filetée, la tige se terminant par une pointe plate de perçage en forme d'ogive.

8. Fiche selon la revendication 1 ou 2, caractérisée en ce que la partie avant dudit tronc de cône est précédée, au moins indirectement, d'un cône comportant la ou les goujures, de manière à réaliser une pointe de perçage.

9. Fiche selon la revendication 8, caractérisée en ce que ledit cône est précédé d'une partie de forme générale cylindrique disposée entre le tronc de cône d'entrée et ledit cône de perçage.

10. Fiche selon la revendication 9, caractérisée en ce que le diamètre de la partie cylindrique de perçage est supérieure au diamètre de fond de filet.

11. Procédé de fabrication d'une fiche transcutanée selon la revendication 1, caractérisé en ce que la goujure est réalisée par meulage après que le filetage avec son tronc de cône d'entrée ait été réalisé.

**Claims**

1. A transcutanerous pin for fixation of a bone part or fragment comprising a threaded portion, the anterior portion of which takes the form of a truncated leading cone, and at least one flute defining a cutting edge extending over said truncated cone of the threading and over the beginning of the central cylindrical portion of said threading, to perform a tapping operation when the end of the threaded portion penetrates the bone part or fragment, characterised in that the flute(s) is(are) disposed helicoidally in relation to the axis of the thread, with the helix and the thread being pitched in the same direction.

2. A pin according to claim 1, characterized in that it includes at least two flutes regularly spaced over the circumference of the pin.

3. A pin according to claim 1 or 2, characterized in that the flute takes the form of a notch formed by two substantially perpendicular surfaces.

4. A pin according to claim 1 or 2, characterized in that the flute takes the form of a notch having a generally rounded shape in cross section.

5. A pin according to claim 1 or 2, characterized in that the anterior portion of said truncated cone is terminated by a face perpendicular to the axis of the pin and intended to fit into a hole previously drilled in the bone part.

6. A pin according to claim 5, characterized in that the edge between said face and the truncated cone is rounded.

7. A pin according to claim 1 or 2, characterized in that the leading truncated cone of the threading is preceded by a rod of diameter equal to or smaller than the inner thread diameter of the threaded portion, said rod terminating in a flat drill tip having the shape of an ogive.

8. A pin according to claim 1 or 2, characterized in that the anterior portion of said truncated cone is preceded, at least indirectly, by a cone including said flute or flutes, so as to form a drill tip.

9. A pin according to claim 8, characterized in that said leading cone is preceded by a portion of generally cylindrical shape located between the truncated leading cone and said drill cone.

10. A pin according to claim 9, characterized in that the diameter of the cylindrical drilling portion is larger than the inner diameter of the thread.

11. Process for manufacturing a transcutaneous pin according to claim 1, wherein he flute is created by grinding after the thread with its leading cone has been made.

**Patentansprüche**

1. Transkutaner Befestigungsstift für Knochenfragmente oder -teile, mit einem Gewindebereich, dessen Vorderbereich einen Einführungs-Kegelstumpf bildet, und mindestens einer sich auf dem Einführungskegel des Gewindes und auf dem Anfang des mittleren, zylindrischen Gewindebereiches befindenden Schneidnut, die eine Schneidkante bildet, zum Schneiden eines Gewindes, wenn das Vorderende des Gewindebereichs in das Knochenfragment bzw. -teil eintritt, dadurch gekennzeichnet, dass sich die Schneidnut bzw. Schneidnuten schraubenförmig um die Stiftachse windet bzw. winden, wobei die Steigung der Schraubung und diejenige des Gewindes gleichsinnig verlaufen.

2. Befestigungsstift nach Anspruch 1, dadurch gekennzeichnet, dass er mindestens zwei über den Stiftumfang regelmässig verteilte Schneidnuten aufweist.

3. Befestigungsstift nach Anspruch 1 oder 2, dadurch gekennzeichnet, dass die Schneidnut als Vertiefung mit zwei aufeinander senkrecht stehenden Flächen ausgebildet ist.

4. Befestigungsstift nach Anspruch 1 oder 2, dadurch gekennzeichnet, dass die Schneidnut als Vertiefung mit abgerundet geformtem Querschnitt ausgebildet ist.

5. Befestigungsstift nach Anspruch 1 oder 2, dadurch gekennzeichnet, dass das Vorderende des genannten Einführungs-Kegelstumpfes eine zur Längsachse des Stiftes senkrechte Ebene ist und das Vorderende dazu bestimmt ist, in eine zuvor im Knochenteil angebrachte Bohrung eingeführt zu werden.

6. Befestigungsstift nach Anspruch 5, dadurch gekennzeichnet, dass der Grat zwischen der genannten Ebene und dem Kegelstumpf abgerundet ist.

7. Befestigungsstift nach Anspruch 1 oder 2, dadurch gekennzeichnet, dass vor dem Einführungskegel des Gewindes eine Verlängerung angebracht ist, deren Durchmesser gleich oder kleiner als der Durchmesser des Gewindekernes ist, und dass die Verlängerung in einer flachen, spitzkegelförmigen Bohrspitze endet.

8. Befestigungsstift nach Anspruch 1 oder 2, dadurch gekennzeichnet, dass mindestens indirekt vor dem Vorderteil des genannten Kegelstumpfes ein Kegel liegt, der die Schneidnut bzw. die Schneidnuten aufweist und dadurch eine Bohrspitze bildet.

9. Befestigungsstift nach Anspruch 8, dadurch gekennzeichnet, dass vor dem genannten Kegel ein zylindrischer Bereich liegt, der sich zwischen dem Einführungs-Kegelstumpf und dem genannten Bohrkegel erstreckt.

10. Befestigungsstift nach Anspruch 9, dadurch gekennzeichnet, dass der Durchmesser des zylindrischen Bohrungsteiles grösser ist als der Durchmesser am Gewindekern.

11. Verfahren zur Herstellung eines transkutanen Befestigungsstifts nach Anspruch 1, dadurch gekennzeichnet, dass die Schneidnut durch Einschleifen angebracht ist, nachdem das Gewinde mit dem Einführungskegel hergestellt worden ist.

FIG. 1

FIG. 2

FIG. 3

FIG. 4

EP 0 230 856 B1

FIG.5

FIG.6

FIG.7

FIG.8

FIG.9

FIG.10